# EUROPEAN PATENT SPECIFICATION

(11) **EP 4 379 042 B1**
(45) Date of publication and mention of the grant of the patent: **06.05.2026**
(21) Application number: 22210213.9
(22) Date of filing: 29.11.2022
(51) Int. Cl.: C12N 5/00

(54) **METHODS FOR THE PREPARATION OF CULTURE MEDIA BASED ON MICROGREENS AND RELATED PRODUCTS**
VERFAHREN ZUR HERSTELLUNG VON KULTURMEDIEN AUF BASIS VON MIKROGRÜNEN UND VERWANDTEN PRODUKTEN
PROCÉDÉS DE PRÉPARATION DE MILIEUX DE CULTURE À BASE DE MICROVERTES ET PRODUITS ASSOCIÉS

(43) Date of publication of application: 05.06.2024
(73) Proprietor: fenaco Genossenschaft, 3001 Bern (CH)
(72) Inventor: Eibl, Regine, Wädenswil (CH); Eibl, Dieter, Wädenswil (CH); Hühn, Tilo, Wädenswil (CH)
(74) Representative: Herrmann, Johanna

(56) References cited:
- CEJAS LUJÁN ET AL: "Malt sprout, an underused beer by-product with promising potential for the growth and dehydration of lactobacilli strains", JOURNAL OF FOOD SCIENCE AND TECHNOLOGY, SPRINGER (INDIA) PRIVATE LTD, INDIA, vol. 54, no. 13, 31 October 2017 (2017-10-31), pages 4464 - 4472, XP036364211, ISSN: 0022-1155, [retrieved on 20171031], DOI: 10.1007/S13197-017-2927-7
- KHALIL SADIA: "EVALUATION OF PLANT BASED PRODUCTS FOR THEIR SUITABILITY AS BACTERIOLOGICAL MEDIA", 1 January 2017 (2017-01-01), XP093042630, Retrieved from the Internet <URL:http://prr.hec.gov.pk/jspui/handle/123456789/11999> [retrieved on 20230427]
- MOURAD ELHUSSEIN F ET AL: "Plant Materials are Sustainable Substrates Supporting New Technologies of Plant-Only-Based Culture Media for <i>in vitro</i> Culturing of the Plant Microbiota", MICROBES AND ENVIRONMENTS, vol. 33, no. 1, 1 January 2018 (2018-01-01), pages 40 - 49, XP093042633, ISSN: 1342-6311, DOI: 10.1264/jsme2.ME17135
- RENNA RENNA MASSIMILIANO MASSIMILIANO ET AL: "Ongoing Research on Microgreens: Nutritional Properties, Shelf-Life, Sustainable Production, Innovative Growing and Processing Approaches", FOODS, 24 June 2020 (2020-06-24), Basel, XP093042664, Retrieved from the Internet <URL:https://mdpi-res.com/books/book/3354/Ongoing_Research_on_Microgreens.pdf?filename=Ongoing_Research_on_Microgreens.pdf> [retrieved on 20230427], DOI: 10.3390/foods9060826
- UPPALA SAI SREE ET AL: "Plant-Based Culture Media for Improved Growth and Sporulation of Cercospora janseana", PLANT DISEASE, vol. 103, no. 3, 1 March 2019 (2019-03-01), US, pages 504 - 508, XP093278125, ISSN: 0191-2917, DOI: 10.1094/PDIS-05-18-0814-RE

## Description

### FIELD OF INVENTION

This invention relates to methods and/or techniques for a cost-effective and sustainable preparation of cell culture media or fermentation media on the basis of microgreens from edible seeds of vegetables, cereals or herbs.

In certain embodiments, the invention relates to the use of microgreens for the preparation of a cell culture medium or fermentation medium, or as a supplement for a cell culture medium or fermentation medium.

### BACKGROUND OF THE INVENTION

Culture media are a source of nutrients and growth factors required for the growth of microorganisms and cells *ex-vivo* under laboratory conditions.

Many types of culture media, which may be classified based on their nutrient composition, consistency or their use in life science laboratories, have been developed to grow selective or desired microorganisms, cells or small plants.

Typically, a general distinction is being made between defined and undefined media.

A defined medium (also known as chemically defined medium or synthetic medium) is a chemically characterised medium produced from pure chemical substances, wherein no yeast, animal, or plant tissue is present. As long as their composition is defined, such media may also be prepared by using purified plant extracts, such as plant proteins (see WO 2021/148 955 A1) or plant peptides and lipids (see WO 98/15614).

On the other hand, an undefined medium (i.e. a basal or complex medium) is characterised in that it contains a carbon source, water, various salts a source of amino acids and nitrogen in unknown quantities. As an example, the addition of organic supplements from natural origin (i.e. coconut water, yeast extract, malt extract, potato extract, banana homogenisate, algal compounds) to prepare undefined plant culture media is reviewed in the publication Z. Molnár et al, Acta Biologica Szegediensis 2011, 55(1), 123-127. US 2011/0212489 A1 discloses a culture medium which comprises a hydrolyzate of plant seeds containing protein and oil (including sunflower seeds). US 2004/0185561 A1 discloses a cell culture medium which comprises a non-hydrolyzed plant extract (e.g. from nuts, peas, or potatoes) and is tested for its growth-promoting effect as part of a cultivation of animal cells, especially keratinocytes. Examples of the use of plant material for the manufacture of culture media are disclosed in Cejas et al., Journal of Food Science and Technology 2017, 54 (13), 4464-4472; S. Khalil "Evaluation of Plant-Based Products for their Suitability as Bacteriological Media", PhD Thesis, University of Karachi, Pakistan, 2017; and Mourad et al. Microbes Environ. 2018, 33 (1), 40-49. An overview of advances in microgreen research is provided in "Ongoing Research on Microgreens: Nutritional Properties, Shelf-Life, Sustainable Production, Innovative Growing and Processing Approaches", M. Paradiso and M. Renna (Eds.) Foods 2020, MDPI.

In commercial cell culture technology, culture media play a very important role in the efficiency and economic viability of production processes at industrial scale. Recent publications suggest that plant cell cultures or their extracts may be used as foodstuffs (see, e.g., E. Nordlund et al., Food Res Int. 2018, 107, 297-305 and R. Eibl et al., Appl. Microbiol. Biotechnol. 2018, 102, 8661-8675) and be manufactured with less energy and lower possible impact on the environment compared to whole plants, and independently of location and season. However, the use of defined cell culture media requires elaborate purification protocols and/or chemicals with a high degree of purity, which is a major cost driver in the transfer of such processes to a commercially relevant scale. While the addition of organic supplements from natural sources (e.g. to prepare undefined plant culture media) may advantageously influence cell survival, adhesion and proliferation, it still remains desirable to effectively produce culture media which contain macronutrients, micronutrients, vitamins, amino acids or nitrogen supplements, source(s) of carbon and growth regulators at reduced processing costs.

Moreover, taking into account that common culture media used in the food science sector are usually derived from pharmaceutical science with a different set of legal requirements for approval, a direct transfer of such technologies to commercial food science for the preparation of cell culture-based food is a major challenge (due to the necessary declaration as novel food, for example). For instance, the addition of hormone supplements for control of cell differentiation may be subject to declaration depending on the application and region.

Hence, it would be desirable to provide culture media for food applications which may be produced sustainably and inexpensively, and which do not solely rely upon chemical *in-vitro* synthesis of such hormone additives.

### SUMMARY OF THE INVENTION

The present invention solves this object with the subject matter of the claims as defined herein. The advantages of the present invention will be further explained in detail in the section below and further advantages will become apparent to the skilled artisan upon consideration of the invention disclosure.

Generally speaking, in one aspect the present invention provides a method for the preparation of a cell culture medium or fermentation medium, comprising: a) growing microgreens from edible seeds of vegetables, cereals or herbs; b) harvesting the microgreens in an immature state; c) grinding the harvested microgreens to an average particle size of 500 µm or less to provide a mash; d) subjecting the ground mash to a phase separation resulting in a solid phase and a liquid phase; e1) optionally adding water to dilute the liquid phase; and f) sterilising the optionally diluted liquid phase to provide the cell culture medium or fermentation medium.

In a second aspect, the present invention relates to a method for the preparation of a supplement for a cell culture medium or fermentation medium, comprising: a) growing microgreens from edible seeds of vegetables, cereals or herbs; b) harvesting the microgreens in an immature state; c) grinding the harvested microgreens to an average particle size of 500 µm or less to provide a mash; d) subjecting the ground mash to a phase separation resulting in a solid phase and a liquid phase; e2) subjecting the liquid phase to a drying and/or concentration step to provide the supplement for a cell culture medium or fermentation medium.

In a third aspect, the present invention relates to the use of microgreens from edible seeds of vegetables, cereals or herbs for the preparation of a cell culture medium or fermentation medium, or as a supplement for a cell culture medium or fermentation medium.

### BRIEF DESCRIPTION OF THE DRAWINGS

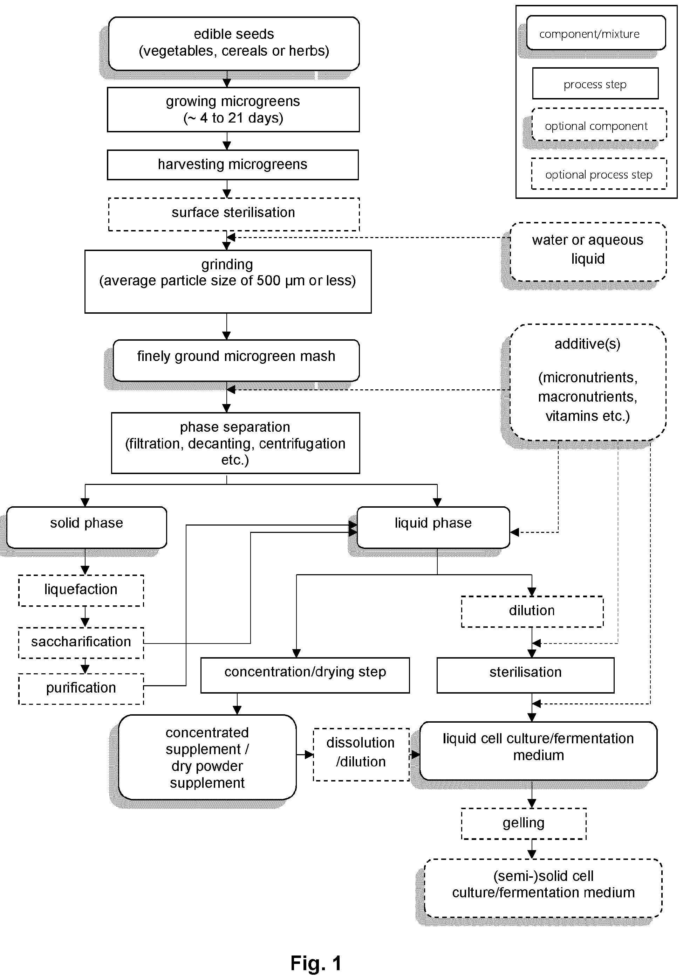

FIG. 1 is a flow chart illustrating examples of the methods according to the first and second embodiments of the present invention.

### DETAILED DESCRIPTION OF THE INVENTION

For a more complete understanding of the present invention, reference is now made to the following description of the illustrative embodiments thereof:

### Methods for the Preparation of Cell Culture or Fermentation Media

In a first embodiment, the present invention generally relates to a method for the preparation of a cell culture medium or fermentation medium, comprising: a) growing microgreens from edible seeds of vegetables, cereals or herbs; b) harvesting the microgreens in an immature state; c) grinding the harvested microgreens to an average particle size of 500 µm or less to provide a mash; d) subjecting the ground mash to a phase separation resulting in a solid phase and a liquid phase; e1) optionally adding water to dilute the liquid phase; and f) sterilising the optionally diluted liquid phase to provide the cell culture medium or fermentation medium.

In a second embodiment, the present invention relates to a method for the preparation of a supplement for a cell culture medium or fermentation medium, comprising: a) growing microgreens from edible seeds of vegetables, cereals or herbs; b) harvesting the microgreens in an immature state; c) grinding the harvested microgreens to an average particle size of 500 µm or less to provide a mash; d) subjecting the ground mash to a phase separation resulting in a solid phase and a liquid phase; e2) subjecting the liquid phase to a drying and/or concentration step to provide the supplement for a cell culture/fermentation medium in concentrated liquid or solid powder form.

Fig. 1 is a flow chart exemplifying a method for the preparation of a cell culture medium or fermentation medium (according to the first embodiment) and a method preparation of a supplement for a cell culture medium or fermentation medium (according to the second embodiment) in accordance with the present invention.

It will be understood that the preferred features specified below with respect to the methods of the invention apply both for the method of the first and second embodiments, except for features that are mutually exclusive.

The terms "culture" or "cell culture", as used herein, refer to the maintenance, growth and/or differentiation of cells in an *in-vitro*-environment.

"Fermentation" as used herein, includes traditional microbial fermentation, where microorganisms convert substrate into edible products, as well as precision fermentation, which involves the production of specific enzymes or protein ingredients, for example, by microorganisms.

"Microgreens", as defined herein, are vegetable, cereal or herb greens that are harvested prior to full growth at the seedling stage (cf. step b)). Specifically, a microgreen is a vegetable green that has been harvested just after the cotyledon leaves have developed, usually when there is just one pair of young "true" leaves. At the harvest stage in step b), which usually takes place 4 to 21 days and preferably 7 to 14 days after germination, microgreens
are typically 1-12 cm (usually 2 - 10 cm) in height, depending on the species. As opposed to sprouts which are produced by germinating seeds that have been soaked in water and for which the edible portion is constituted by the whole sprout including the seedling, the radicles, and often what remains of the seeds, the edible portion of microgreens is usually constituted by single shoots with the cotyledon leaves and/or the first true leaves which are preferably harvested cutting the young seedlings at the base right above the growing medium.

Microgreens may be grown on large or small scales in any environment, and once the growth phase has been initiated, the process does not require much attention or expenditure on part of the manufacturer, thus enabling simple and inexpensive on-site production of culture media. In addition, the use of microgreens allow for a sustainable production of useful micro- and macronutrients for complex culture media by using regenerative or biological agriculture. While not being limited thereto, commercially available growing kits may be employed to further simplify the growing operation, which typically include a (fibrous) seed mat with nutrients and seeds. Examples thereof are disclosed in WO 2015/110554 A1, US 2017/0172082 A1 and US 2021/0345562 A1.

Advantageously, microgreens characteristically contain high concentrations of carbohydrates, amino acids, proteins, minerals, antioxidants, vitamins and/or plant growth hormones, and thus provide an excellent basis for culture media. For instance, Z. Xiao et al., J. Agric. Food Chem. 2012, 60, 7644-7651 discloses based on the analysis of 25 species that microgreens, on average, have four to six times more nutrients than their mature counterparts. Accordingly, the methods of the present invention are not only significantly less expensive than chemical synthesis but also do not necessarily require a declaration for legal approval, since no additional hormone supplements are required. In addition, it has also been shown that microgreens are microbiologically safer than sprouts, as they are harvested without roots and seed coats and, therefore, are less likely to be exposed to contamination in the growing media and the seed itself.

Depending on the selection of edible seeds of vegetables, cereals or herbs, the microgreens may be grown under various lighting conditions (e.g. including indirect ambient light, sunlight and/or artificial light) or in complete darkness, which - in combination with the choice and concentration of nutrient addition as well as the temperature and humidity conditions during the microgreen growth phase - further offers the opportunity to modify the concentration of antioxidants and other bioactive compounds in the final culture medium. For instance, in some cases, UV and blue light as well as high light exposure may be effectively used to promote biosynthesis of such compounds.

In general, the selection of edible seeds of vegetables, cereals or herbs for microgreen production is not particularly limited. Examples of vegetables and herbs include, but are not limited to the *Amaranthaceae* family (including, e.g., amaranth, quinoa swiss chard, beet and spinach), *Amaryllidaceae* family (including, e.g., garlic, onion and/or leek), *Apiaceae* family (including, e.g., dill, carrot, fennel and/or celery), *Asteraceae* family (including, e.g., lettuce, endive, chicory and/or radicchio), *Brassicaceae* family (including, e.g., cauliflower, broccoli, cabbage, watercress, radish and/or arugula), *Cucurbitaceae* family (including, e.g., melon, cucumber and squash) and legumes (e.g. chickpeas, lentils and/or beans). Examples of cereals include, but are not limited to rice, oats, wheat, corn and barley. It should be considered that the optimum concentration of each nutrient for achieving maximum growth rates varies among species. In this regard, the growth conditions and post-harvest processes may be suitably adapted by the skilled artisan to each microgreen species to achieve the desired nutrient profile.

In this respect, step a) according to the methods of the present invention preferably comprises growing microgreens of corn (*Zea mays*) and further preferably sweet corn (*Zea mays convar. saccharata var. rugosa*), since it enables microgreen growth under dark and humid conditions, thereby eliminating variations inherent to natural light exposure and/or increased energy expenditure due to controlled artificial light exposure. Moreover, sweet corn microgreens contain especially high sugar (e.g. sucrose and glucose) concentrations, which eliminates the need for a saccharification step (further described below), which further simplifies the manufacturing process.

Notably, multiple types of microgreens may be combined to further fine-tune the nutrient composition in the produced media.

In step b), the microgreens are harvested, e.g. by cutting the young seedlings at the base right above the growing medium, which usually takes place 4 to 21 days and more preferably 7 to 14 days after germination.

Before the grinding step c), the harvested microgreens may be preferably washed and/or surface-sterilised. The washing and/or surface sterilisation steps are not particularly limited as long as they effectively reduce microbial surface contamination, and suitable methods may include chemical sterilisation (e.g., with ethanol solution (≥ 70% (v/v), 8-hydroxyquinoline and its derivatives or salts thereof, and sodium hypochlorite solution (e.g. 5% (v/v)) or the like), washing in sterile water, surface sterilisation or pasteurisation by means of heat and/or irradiation with ionizing rays (e.g. UV irradiation), ultrasound-based sterilisation and combinations thereof. In preferred embodiments, surface sterilisation is brought about by saturated steam pasteurisation in partial vacuum.

Thereafter, in step c), the harvested microgreens are ground to an average particle size of 500 µm or less. Average particle sizes of 200 µm or less, 100 µm or less, 50 µm or less or even 30 µm or less are further preferred to enhance the release of nutrients. The grinding of the mixture may be carried out in one or in multiple steps. The mill used for this purpose is not particularly limited and may include a pin mill, a hammer mill, a screen mill, a colloid mill or combinations thereof (e.g. in case of multiple grinding steps). The use of mills operating by use of vibration, oscillation and/or rotation at ultrasonic frequencies (such as e.g. a tooth colloid mill) in least one grinding (sub-)step may be preferable as it advantageously affects the extraction efficiency, presumably due to sonochemically induced reaction dynamics. In general, the average particle size may be measured as volume moment mean (*D*[4,3]), and is typically defined by all particle sizes contributing according to their volume fraction in the collective, so that the average particle size in the interval is weighted with the corresponding volume portion and all these weighted values are averaged arithmetically. The particle sizes and their distribution may be suitably determined by methods known in the art (e.g. by a particle size analyzer).

In a preferred embodiment, the grinding step c) is carried out at a temperature lower than 65°C, more preferably lower than 40°C and especially preferably at temperatures of 0 to 35°C, such as e.g. lower or equal to ambient temperature (i.e. 25°C). In another preferred embodiment, the harvested microgreen may be subjected to wet grinding, more preferably in the presence of water, which may be optionally cooled before being added so as to further control the temperature regime. Such methods enable a high release of microgreen constituents into the liquid phase while avoiding degradation of thermally sensitive antioxidants, vitamins and other biologically active agents.

In step d), the finely ground mash is subjected to a phase separation resulting in a solid phase and a liquid phase. The method of separating the phases is not particularly limited and may, for example, include filtration, decanting, centrifugation, or the like.

In a preferred embodiment, the solid phase is processed independently from the liquid phase and recombined with the liquid phase before step e1) or e2).

As an example, independent processing of the solid phase may include a step of filtering, centrifuging or heating (e.g. under vacuum) the initially separated solid phase to further remove remaining liquids, and to recombine said liquid phase with the water phase from the initial separation step or at a later processing stage of said phases. Also, the liquid phase may be subjected to further purification steps, e.g. by membrane processes or filtration using vacuum rotation filters in order to remove fine particles.

In a preferred embodiment, the solid phase is independently processed by subjecting the same to a saccharification step, wherein carbohydrate-containing materials (e.g., starchy materials, cellulosic materials, lignocellulosic materials) are converted to low molecular weight sugars, such as sucrose and/or glucose, for example.

In a preferred embodiment, the saccharification step includes an enzymatic treatment, acidic treatment, solid catalyst-assisted microwave irradiation, thermal treatment under pressure, steam treatment or combinations thereof.

Independently or in combination, the solid phase may be subjected to a liquefaction step prior to the saccharification step.

In a preferred embodiment, liquefaction may be accomplished by slurrying the solid phase in water, gelatinising the slurry and hydrolyzing the same in the presence of one or more types of starch-hydrolyzing enzymes. Examples of starch-hydrolyzing enzymes include, but are not limited to α-amylase and amyloglucosidase. Suitable conditions for liquefaction may be appropriately selected by the skilled artisan. A non-limiting, exemplary method for liquefaction is disclosed in DE102004026152 A1 , for example. The thus obtained hydrolyzed slurry may be optionally further processed and recombined with the liquid phase to provide the cell culture or fermentation medium.

Notably, the separation of the liquid phase from the solid phase in step d) has the advantage that the liquid phase remains unaffected by the processing steps applied to the separated solid phase. Accordingly, the liquid phase does not need to be subjected to harsh processing conditions which may apply during saccharification, so that heat-sensitive micro- and macronutrients transferred to the liquid phase during grinding and mixing may be preserved.

In this respect, it is preferred that during the entire preparation process, the liquid phase is not subjected to temperatures of 55°C or above, preferably 45°C or above. Alternatively or in combination, only the solid phase is subjected to a thermal treatment at 65°C or above, such as 85°C or higher.

While not being necessary *per se,* further additives may be added to the mash or the liquid phase during or after step c) in order to further adjust, enrich and/or complement the nutrient profile according to the desired application. While not being limited thereto, such additives may comprise macronutrients (e.g., sources of nitrogen (N), phosphorus (P), potassium (K), calcium (Ca), magnesium (Mg) and sulphur (S)), micronutrients (e.g. sources of iron (Fe), manganese (Mn), zinc (Zn), boron (B), copper (Cu) and molybdenum (Mo)), source(s) of carbon (e.g., sucrose, glucose, sugar cane molasses, banana extract and coconut water), vitamins (e.g., thiamin (B1), nicotinic acid, pyridoxine (B6), biotin, folic acid, ascorbic acid, pantothenic acid, tocopherol (vitamin E), riboflavin, p-amino- and benzoic acid), amino acids or nitrogen supplements (e.g., casein hydrolysate, L-glutamine, L-asparagine, adenine, glycine, glutamine, asparagine, L-arginine, cysteine and L-tyrosine), undefined organic supplements (e.g., protein hydrolysates, coconut milk, yeast extract, malt extract, ground banana, orange juice and tomato juice), growth regulators (e.g. natural and synthetic auxins, cytokinins, gibberellins, abscisic acid and derivatives thereof) and solidifying agents. Preferred additives include, but are not limited to: one or more salts including Ca²⁺, SO₄²⁻, Mg²⁺ , PO₄³⁻, Cl⁻, NO₃⁻ and/or NH₄⁺ ions; one or more vitamins, preferably Vitamin B5; one or more growth regulators, preferably one or more selected from sterile coconut water, a cytokinin and an auxin, more preferably one or more selected from thidiazuron, zeatin or kinetin gibberellic acid, 2,4-dichlorophenoxyacetic acid (2,4-D), indoleacetic acid (IAA) or indolebutyric acid (IBA); and/or one or more phytohormones.

Once being processed according to the above description, the liquid phase (with or without prior recombination of the liquified saccharified extract obtained from the solid phase) may be diluted in water (preferably sterile water) in step e1), if necessary, and sterilised to provide the cell culture or fermentation medium in ready-to-use form in step f).

The method of sterilisation is not particularly limited and may include a heat treatment (e.g., autoclaving), irradiation with ionising rays, ultrasound-based sterilisation, sterile filtration through microporus filters and combinations thereof, while sterile filtration is preferably used in the presence of thermolabile constituents.

A solifidying agent (e.g., agar) may be added the liquid cell culture or fermentation medium at a concentration of 0.1 to 0.5 % wt.-% or 1 to 3.0 wt.-% to provide a semi-solid or solid medium, respectively.

Another convenient method for preparation of cell culture or fermentation media is to prepare a supplement in form of a dry powder or a concentrated stock solution, which can be be dissolved and/or diluted to the preferred concentration immediately before use, respectively. In this case, the liquid phase obtained in step d) (with or without prior recombination of the liquified saccharified extract obtained from the solid phase) is subjected to a concentration and/or drying step in step e2). Such a step may involve thermal treatment under low pressure conditions to expel water. However, low-temperature methods, such as freeze-drying, are preferred due to reduced loss of heat-sensitive nutrients.

### Media and Media Supplements

According to the invention a cell culture medium or fermentation medium may be obtained by the method according to the first embodiment.

As outlined above, the cell culture medium or fermentation medium obtained by method according to the invention contains favorable concentrations of macronutrients, micronutrients, vitamins, amino acids or nitrogen supplements, source(s) of carbon and growth regulators, may be produced sustainably and inexpensively by regenerative or biological agriculture, and does not necessitate elaborate purification protocols in comparison to defined media.

In addition, a supplement for a cell culture medium or fermentation medium may be obtained by the method according to second embodiment.

Here, the supplement may be provided in powder form or as a concentrate, which may be stored in a refrigerator at 2 - 4°C or in a freezer (-20°C), respectively. It will be understood that concentrated stock solutions and powders produced according to the aforementioned method need not to be used as constituent media upon dilution, but may also be employed as additive for known cell culture media or fermentation media.

### Use of Microgreens for the Preparation of Cell Culture or Fermentation Media

As stated above, microgreens provide a unique set of advantages in the preparation of cell culture or fermentation media, since they enable simple upscaling, inexpensive and sustainable production and further provide high contents of micro- and macronutrients for complex culture media.

Therefore, in a third embodiment, the present invention relates to the use of microgreens from edible seeds of vegetables, cereals or herbs for the preparation of a cell culture medium or fermentation medium, or as a supplement for a cell culture medium or fermentation medium.

## Claims

1. Method for the preparation of a cell culture medium or fermentation medium, comprising:
a) growing microgreens from edible seeds of vegetables, cereals or herbs;
b) harvesting the microgreens in an immature state;
c) grinding the harvested microgreens to an average particle size of 500 µm or less to provide a mash;
d) subjecting the ground mash to a phase separation resulting in a solid phase and a liquid phase;
e1) optionally adding water to dilute the liquid phase; and
f) sterilising the optionally diluted liquid phase to provide the cell culture medium or fermentation medium.

2. Method for the preparation of a supplement for a cell culture medium or fermentation medium, comprising:
a) growing microgreens from edible seeds of vegetables, cereals or herbs;
b) harvesting the microgreens in an immature state;
c) grinding the harvested microgreens to an average particle size of 500 µm or less to provide a mash;
d) subjecting the ground mash to a phase separation resulting in a solid phase and a liquid phase;
e2) subjecting the liquid phase to a drying and/or concentration step to provide the supplement for a cell culture medium or fermentation medium.

3. Method according to any one of claims 1 or 2, wherein step a) comprises growing microgreens of corn (*Zea mays*)*,* preferably sweet corn (*Zea mays convar. saccharata var. rugosa*), under dark and humid conditions.

4. Method according to any of claims 1 to 3, wherein the solid phase is processed independently from the liquid phase and recombined with the liquid phase before step e1) or e2).

5. Method according to claim 4, wherein the solid phase is independently processed by subjecting the same to a saccharification step.

6. Method according to claim 5, wherein the saccharification step includes an enzymatic treatment, acidic treatment, solid catalyst-assisted microwave irradiation, thermal treatment under pressure, steam treatment or combinations thereof.

7. Method according to any of claims 5 or 6, wherein the solid phase is subjected to a liquefaction step prior to the saccharification step, preferably by slurrying the solid phase in water, gelatinising the slurry and hydrolyzing the same in the presence of one or more types of starch hydrolyzing enzymes.

8. Method according to any of claims 1 to 7, wherein the harvested microgreens are subjected to wet grinding in step c), preferably in the presence of water.

9. Method according to any of claims 1 to 8, wherein step c) is performed at temperatures below 65°C, preferably below 40°C, more preferably at temperatures of 0 to 35°C.

10. Method according to any of claims 1 to 9, wherein in step c), the harvested microgreens are ground to an average particle size of 100 µm or less, preferably 50 µm or less, more preferably 30 µm or less.

11. Method according to any of claims 1 to 10, wherein during the entire preparation process, the liquid phase is not subjected to temperatures of 55°C or above, preferably 45°C or above, and/or only the solid phase is subjected to a thermal treatment at 65°C or above, such as 85°C or higher.

12. Method according to any of claims 1 to 11, wherein one or more of the following is further added during or after step c):
one or more salts including Ca²⁺, SO₄²⁻, Mg²⁺, PO₄³⁻, Cl⁻, NO₃⁻ and/or NH₄⁺ ions;
one or more vitamins, preferably Vitamin B5;
one or more growth regulators, preferably one or more selected from sterile coconut water, a cytokinin and an auxin, more preferably one or more selected from thidiazuron, zeatin or kinetin gibberellic acid, 2,4-dichlorophenoxyacetic acid (2,4-D), indoleacetic acid (IAA) or indolebutyric acid (IBA); and/or
one or more phytohormones.

13. Use of microgreens from edible seeds of vegetables, cereals or herbs for the preparation of a cell culture medium or fermentation medium, or as a supplement for a cell culture medium or fermentation medium.

## Patentansprüche

1. Verfahren zur Herstellung eines Zellkulturmediums oder Fermentationsmediums, umfassend:
a) Anbauen von Mikrogrünen aus essbaren Samen von Gemüse, Getreide oder Kräutern;
b) Ernten der Mikrogrüne in einem unreifen Zustand;
c) Mahlen der geernteten Mikrogrüne auf eine durchschnittliche Partikelgröße von 500 µm oder weniger, um eine Maische zu erhalten;
d) Unterziehen der gemahlenen Maische einer Phasentrennung, die zu einer festen Phase und einer flüssigen Phase führt;
e1) optionales Hinzufügen von Wasser, um die flüssige Phase zu verdünnen; und
f) Sterilisieren der optional verdünnten flüssigen Phase, um das Zellkulturmedium oder Fermentationsmedium zu erhalten.

2. Verfahren zur Herstellung eines Zusatzstoffes für ein Zellkulturmedium oder Fermentationsmedium, umfassend:
a) Anbauen von Mikrogrünen aus essbaren Samen von Gemüse, Getreide oder Kräutern;
b) Ernten der Mikrogrüne in einem unreifen Zustand;
c) Mahlen der geernteten Mikrogrüne auf eine durchschnittliche Partikelgröße von 500 µm oder weniger, um eine Maische zu erhalten;
d) Unterziehen der gemahlenen Maische einer Phasentrennung, die zu einer festen Phase und einer flüssigen Phase führt;
e2) Unterziehen der flüssigen Phase einem Trocknungs- und/oder Konzentrationsschritt, um den Zusatzstoff für ein Zellkulturmedium oder Fermentationsmedium bereitzustellen.

3. Verfahren nach einem der Ansprüche 1 oder 2, wobei Schritt a) das Züchten von Mikrogrünen aus Mais (*Zea mays*), vorzugsweise Zuckermais (*Zea mays convar. saccharata var. rugosa*), unter dunklen und feuchten Bedingungen umfasst.

4. Verfahren nach einem der Ansprüche 1 bis 3, wobei die feste Phase unabhängig von der flüssigen Phase verarbeitet und vor Schritt e1) oder e2) wieder mit der flüssigen Phase rekombiniert wird.

5. Verfahren nach Anspruch 4, wobei die feste Phase unabhängig verarbeitet wird, indem sie einem Verzuckerungsschritt unterzogen wird.

6. Verfahren nach Anspruch 5, wobei der Verzuckerungsschritt eine enzymatische Behandlung, eine Säurebehandlung, eine mit einem festen Katalysator unterstützte Mikrowellenbestrahlung, eine thermische Behandlung unter Druck, eine Dampfbehandlung oder Kombinationen davon umfasst.

7. Verfahren nach einem der Ansprüche 5 oder 6, wobei die feste Phase vor dem Verzuckerungsschritt einem Verflüssigungsschritt unterzogen wird, vorzugsweise durch Aufschlämmen der festen Phase in Wasser, Gelatinieren der Aufschlämmung und Hydrolysieren derselben in Gegenwart einer oder mehrerer Arten von stärkehydrolysierenden Enzymen.

8. Verfahren nach einem der Ansprüche 1 bis 7, wobei die geernteten Mikrogrüne in Schritt c) einer Nassmahlung unterzogen werden, vorzugsweise in Gegenwart von Wasser.

9. Verfahren nach einem der Ansprüche 1 bis 8, wobei Schritt c) bei Temperaturen unter 65 °C, vorzugsweise unter 40 °C, noch bevorzugter bei Temperaturen von 0 bis 35 °C durchgeführt wird.

10. Verfahren nach einem der Ansprüche 1 bis 9, wobei in Schritt c) die geernteten Mikrogrüne auf eine durchschnittliche Partikelgröße von 100 µm oder weniger, vorzugsweise 50 µm oder weniger, noch bevorzugter 30 µm oder weniger gemahlen werden.

11. Verfahren nach einem der Ansprüche 1 bis 10, wobei während des gesamten Herstellungsprozesses die flüssige Phase keinen Temperaturen von 55 °C oder darüber, vorzugsweise 45 °C oder darüber, ausgesetzt wird und/oder nur die feste Phase einer thermischen Behandlung bei 65 °C oder darüber, beispielsweise 85 °C oder höher, unterzogen wird.

12. Verfahren nach einem der Ansprüche 1 bis 11, wobei während oder nach Schritt c) zusätzlich eines oder mehrere der folgenden Mittel zugegeben werden:
ein oder mehrere Salze, die Ionen ausgewählt aus Ca²⁺, SO₄²⁻, Mg²⁺, PO₄³⁻, Cl⁻, NO₃⁻ und/oder NH₄⁺⁻ enthalten;
ein oder mehrere Vitamine, vorzugsweise Vitamin B5;
ein oder mehrere Wachstumsregulatoren, vorzugsweise ein oder mehrere, ausgewählt aus sterilem Kokosnusswasser, einem Cytokinin und einem Auxin, noch bevorzugter ein oder mehrere, ausgewählt aus Thidiazuron, Zeatin oder Kinetin, Gibberellinsäure, 2,4-Dichlorphenoxyessigsäure (2,4-D), Indolessigsäure (IAA) oder Indolbuttersäure (IBA); und/oder
ein oder mehrere Phytohormone.

13. Verwendung von Mikrogrünen aus essbaren Samen von Gemüse, Getreide oder Kräutern zur Herstellung eines Zellkulturmediums oder Fermentationsmediums oder als Zusatzstoff für ein Zellkulturmedium oder Fermentationsmedium.

## Revendications

1. Procédé de préparation d'un milieu de culture cellulaire ou d'un milieu de fermentation, comprenant :
a) la culture de micro-pousses à partir de graines comestibles de légumes, de céréales ou d'herbes ;
b) la récolte des micro-pousses à un stade immature ;
c) le broyage des micro-pousses récoltées jusqu'à obtenir une taille moyenne de particules de 500 µm ou moins afin d'obtenir une purée ;
d) la soumission de la purée broyée à une séparation de phases résultant en une phase solide et une phase liquide ;
e1) éventuellement l'ajout de l'eau pour diluer la phase liquide ; et
f) la stérilisation de la phase liquide éventuellement diluée pour obtenir le milieu de culture cellulaire ou le milieu de fermentation.

2. Procédé de préparation d'un supplément pour un milieu de culture cellulaire ou un milieu de fermentation, comprenant :
a) la culture de micro-pousses à partir de graines comestibles de légumes, de céréales ou d'herbes ;
b) la récolte des micro-pousses à un stade immature ;
c) le broyage des micro-pousses récoltées jusqu'à obtenir une taille moyenne de particules de 500 µm ou moins afin d'obtenir une purée ;
d) la soumission de la purée broyée à une séparation de phases résultant en une phase solide et une phase liquide ;
e2) la soumission de la phase liquide à une étape de séchage et/ou de concentration pour obtenir le supplément pour un milieu de culture cellulaire ou un milieu de fermentation.

3. Procédé selon l'une quelconque des revendications 1 ou 2, dans lequel l'étape a) comprend la culture de micro-pousses de maïs (*Zea mays*), de préférence de maïs sucré (*Zea mays convar. saccharata var. rugosa*), dans des conditions sombres et humides.

4. Procédé selon l'une quelconque des revendications 1 à 3, dans lequel la phase solide est traitée indépendamment de la phase liquide et recombinée avec la phase liquide avant l'étape e1) ou e2).

5. Procédé selon la revendication 4, dans lequel la phase solide est traitée indépendamment en la soumettant à une étape de saccharification.

6. Procédé selon la revendication 5, dans lequel l'étape de saccharification comprend un traitement enzymatique, un traitement acide, une irradiation par micro-ondes assistée par un catalyseur solide, un traitement thermique sous pression, un traitement à la vapeur ou des combinaisons de ceux-ci.

7. Procédé selon l'une quelconque des revendications 5 ou 6, dans lequel la phase solide est soumise à une étape de liquéfaction avant l'étape de saccharification, de préférence en mettant en suspension la phase solide dans de l'eau, en gélatinisant la suspension et en l'hydrolysant en présence d'un ou plusieurs types d'enzymes d'hydrolyse de l'amidon.

8. Procédé selon l'une quelconque des revendications 1 à 7, dans lequel les micro-pousses récoltées sont soumises à un broyage humide à l'étape c), de préférence en présence d'eau.

9. Procédé selon l'une quelconque des revendications 1 à 8, dans lequel l'étape c) est réalisée à des températures inférieures à 65 °C, de préférence inférieures à 40 °C, de préférence encore à des températures comprises entre 0 et 35 °C.

10. Procédé selon l'une quelconque des revendications 1 à 9, dans lequel, à l'étape c), les micro-pousses récoltées sont broyées jusqu'à obtenir une taille moyenne de particules de 100 µm ou moins, de préférence 50 µm ou moins, et plus préférablement 30 µm ou moins.

11. Procédé selon l'une quelconque des revendications 1 à 10, dans lequel, pendant tout le processus de préparation, la phase liquide n'est pas soumise à des températures de 55 °C ou plus, de préférence de 45 °C ou plus, et/ou seule la phase solide est soumise à un traitement thermique à 65 °C ou plus, par exemple à 85 °C ou plus.

12. Procédé selon l'une quelconque des revendications 1 à 11, dans lequel un ou plusieurs des éléments suivants sont en outre ajoutés pendant ou après l'étape c) :
un ou plusieurs sels comprenant des ions Ca²⁺ , SO₄²⁻, Mg²⁺ , PO₄³⁻ , Cl⁻ , NO₃⁻ et/ou NH₄⁺ ;
une ou plusieurs vitamines, de préférence la vitamine B5 ;
un ou plusieurs régulateurs de croissance, de préférence un ou plusieurs choisis parmi l'eau de coco stérile, une cytokinine et une auxine, de préférence un ou plusieurs sélectionnés parmi le thidiazuron, la zéatine ou la kinétine, l'acide gibbérellique, l'acide 2,4-dichlorophénoxyacétique (2,4-D), l'acide indoleacétique (IAA) ou l'acide indolebutyrique (IBA) ; et/ou
une ou plusieurs phytohormones.

13. Utilisation de micro-pousses issues de graines comestibles de légumes, de céréales ou d'herbes aromatiques pour la préparation d'un milieu de culture cellulaire ou d'un milieu de fermentation, ou comme complément à un milieu de culture cellulaire ou à un milieu de fermentation.
